# EUROPÄISCHE PATENTANMELDUNG

(11) **EP 4 321 107 A1**
(43) Veröffentlichungstag der Anmeldung: **14.02.2024**
(21) Anmeldenummer: 23189302.5
(22) Anmeldetag: 02.08.2023
(51) Int. Cl.: A61B 17/16

(54) **MEDIZINISCHES MOTORHANDSTÜCK MIT RAST- UND/ODER ANSCHLAGEINHEIT**

(30) Priorität: 09.08.2022 DE 102022119980
(71) Anmelder: Aesculap AG, 78532 Tuttlingen (DE)
(72) Erfinder: BUERK, Andre, 78056 Villingen-Schwenningen (DE); VOGLER, Aaron, 88637 Leibertingen (DE)
(74) Vertreter: Winter, Brandl - Partnerschaft mbB

(57) **Zusammenfassung**

Die Offenbarung betrifft ein medizinisches Motorhandstück (2) zum Antreiben eines distalen Endeffektors (32), mit einem Handgriffabschnitt (4), einem vorzugsweise hülsenförmigen Bedienelement (12), das um eine Handgrifflängsachse drehbar an einem distalen Endabschnitt des Handgriffabschnitts (4) gehalten ist und mit dem Endeffektor (32) derart koppelbar ist, um eine Drehbewegung des Bedienelements (12) in eine Bewegung des Endeffektors (32) zu transformieren oder eine Funktion am Endeffektor (32) zu bewirken und eine Rast- und/oder Anschlageinheit, die dafür vorgesehen und ausgebildet ist, die Drehbewegung des Bedienelements (12) an Endpositionen zu begrenzen und/oder einen Drehbewegungswiderstand auf das Bedienelement (12) in wenigstens einer Drehzwischenposition zu erhöhen.

Die Rast- und/oder Anschlageinheit weist zumindest einen Schraubstift (88; 112), welcher längs zur Handgrifflängsachse sich erstreckend in ein Einstellgewinde (108, 110) des Bedienelements (112) eingeschraubt ist, und eine Abgleitkulisse (94) auf, die drehfest am Handgriffabschnitt (4) gehalten und mit dem zumindest einen Schraubstift (88; 112) in Gleiteingriff bringbar ist.

## Beschreibung

Die Offenbarung betrifft ein medizinisches Motorhandstück mit einer Rast-und/oder Anschlageinheit sowie ein medizinisches Handinstrument mit dem medizinischen Motorhandstück.

### Hintergrund der Offenbarung

In der modernen minimalinvasiven Chirurgie werden derartige Werkzeuge/Instrumente und dazugehörige Instrumentenhandstücke/Handinstrumente beispielsweise zur Bearbeitung von Knochen, Knorpeln bei arthroskopischen Eingriffen, in der Wirbelsäulenchirurgie und dergleichen orthopädischen/chirurgischen Behandlungen sowie zur Bearbeitung von organischem Material in der Neurochirurgie verwendet. Die Werkzeuge/Instrumente weisen ein(en) Handstück/Handgriff/Griffabschnitt und ggf. austauschbare Effektoren, wie beispielsweise Fräser, Drehmesser, einen Polierkopf oder Ähnliches auf. Der Effektor ist in einem Schaft des Werkzeugs/Instruments an dessen distalem Ende gelagert, ggf. drehbar angetrieben gelagert. Als Werkzeugantrieb ist je nach Verwendungszweck und beabsichtigter Werkzeugdrehzahl ein hydraulischer, pneumatischer, oder elektromotorischer Antrieb vorgesehen, der über einen Drehmoment-Übertragungszug innerhalb des Werkzeugs und/ oder des Handinstruments bzw. des Griffabschnitts mit dem Werkzeugkopf (Effektor) wirkverbunden ist. Die Antriebe können dabei im Werkzeug und/oder im Handinstrument integriert oder als externe Antriebseinheiten ausgebildet sein, die über Energieversorgungsleitungen oder Drehmoment-Übertragungsstränge mit dem Werkzeug oder dem Handinstrument gekuppelt sind.

Dabei ist es vorteilhaft, den distalen Schaftabschnitt eines Schafts von einem medizinischen Handinstrument abzuwinkeln, um so Operationen in geringem Raum durchführen zu können, beispielsweise bei Operationen an der Wirbelsäule. Anders ausgedrückt, spielt bei chirurgischen, insbesondere minimal-invasiven, Eingriffen der Bauraum der verwendeten Instrumente und eine gute Handhabbarkeit eine große Rolle. So sollen die Instrumentenschäfte insbesondere im Bereich der distalen Effektoren auf möglichst kleinem Raum aktiv (über einen Betätigungsmechanismus gewollt ausgeführt) abwinkelbar sein.

Hierbei ist von großer Bedeutung, dass der Betätigungsmechanismus durch einen Bediener intuitiv und sicher bedient werden kann, auch ohne dass der Bediener seinen Blick von einer Eingriffsstelle des Effektors abwendet, um eine Patientensicherheit zu erhöhen. Anders ausgedrückt ist es wünschenswert, dass der Bediener den Betätigungsmechanismus blind bedienen kann.

### Stand der Technik

Abwinkelbare Schäfte für medizinische Handinstrumente sind hinlänglich bekannt und weisen üblicherweise einen proximalen und einen abwinkelbaren distalen Schaftabschnitt auf. Der distale Schaftabschnitt und der proximale Schaftabschnitt weisen dabei beide jeweils ein schräges Ende/ eine bezüglich der jeweiligen Schaftabschnittsachse angestellte Stirnseite auf. D.h. jeweils ein Ende/ Endabschnitt/ Stirnseite des distalen und proximalen Schaftabschnitts ist nicht gerade, sondern abgeschrägt/ angestellt. Die Schrägungen/ angestellten Endabschnitte/ Stirnseiten haben jeweils den im Wesentlichen gleichen Anstellwinkel. Darum passen die Schrägungen derart zueinander, dass der proximale und der distale Schaftabschnitt in einer bestimmten Relativdrehposition einen geraden Schaft/ ein gerades Rohr bilden. Wenn nun der distale Schaftabschnitt um seine Längsachse relativ zum proximalen Schaftabschnitt rotiert und der proximale Schaftabschnitt stehen bleibt, wird der distale Schaftabschnitt durch die angestellten Stirnseiten/ Endabschnitte zwangsläufig abgewinkelt.

Beispielsweise sind in US 7,585,300 B2 oder US 10,070,872 B2 Beispiele für chirurgische Instrumente mit einem Handstück und einem in dem Handstück aufgenommenen Schaft, an dessen distalen Ende ein Werkzeugkopf schwenkbar angelenkt ist, offenbart. Die Verschwenkung des Werkzeugkopfs kann dabei über ein an dem Handstück drehbar angeordnetes Handrad bewirkt werden. Ähnlich dazu, ist in US 8,303,594 B2 ein chirurgisches Handstück offenbart, bei welchem die Verschwenkung des Werkzeugkopfs über einen an dem Handstück angeordneten Hebel bewirkt wird.

Darüber hinaus zeigt US 9,597,093 B2 ein Werkzeug, welches mit seinem proximalen Ende drehmomentübertragend mit einer Antriebseinheit gekoppelt werden kann und an seinem distalen Ende einen Werkzeugkopf aufweist. Der Werkzeugkopf kann durch Rotation einer in dem Bereich des Werkzeugkopfs angeordneten Hülse relativ zu einem Werkzeugschaft verschwenkt werden.

Ferner offenbart auch DE 10 2017 010 033 A1 eine medizinische Vorrichtung mit einer Führungseinheit, die ein Führungsrohr mit einer Längsachse, ein fest mit diesem verbundenes proximales erstes Kopplungsteil und distal einen zylindermantelförmigen Schwenkkopf aufweist, sowie mit einem im Führungsrohr axial beweglichen, mit dem Schwenkkopf verbundenen Betätigungsrohr, das durch ein proximales Bedienungselement ein Verschwenken des Schwenkkopfes bewirkt. Zur präziseren Ausrichtung eines distalen Führungselements für ein drehbares chirurgisches Werkzeug und damit der winkelmäßigen Ausrichtung des Arbeitskopfes eines solchen Werkzeugs ist das Bedienungselement um die Längsachse verschwenkbar und bewirkt unter Axialverschiebung des Betätigungsrohrs das Verschwenken des Schwenkkopfes. Das Bedienelement ist mit einer über eine Feder vorgespannten Kugel ausgebildet, welche in Positionen bestimmter Winkelstellungen einrasten kann.

Der Stand der Technik hat jedoch immer den Nachteil, dass das Bedienelement entweder keine Raststufen für verschiedene Winkelstellungen aufweist und somit kein haptisches Feedback an einen Bediener/Verwender ausgibt oder, wie in der DE 10 2017 010 033 A1 zwar Raststufen ausgebildet sind, welche dem Bediener ein haptisches Feedback ausgeben, eine mit einer Feder vorgespannte Kugel jedoch aufwendig in eine innenliegende Bohrung eingebracht werden muss und ein Einstellen eines zu überwindenden Feedbackwiderstandes mit erhöhtem Demontage- und anschließendem Montageaufwand verbunden ist.

### Zusammenfassung der Offenbarung

Es sind die Aufgaben und Ziele der Offenbarung, die Nachteile aus dem Stand der Technik zu beheben oder wenigstens zu mindern und insbesondere ein medizinisches Motorhandstück bereitzustellen, dessen Bedienelement einem Bediener ein haptisches Feedback ausgibt und dabei leicht zu montieren und zu justieren ist.

Die Aufgaben und Ziele werden hinsichtlich eines gattungsgemäßen medizinischen Motorhandstücks offenbarungsgemäß durch den Gegenstand des Anspruchs 1 sowie hinsichtlich eines gattungsgemäßen medizinisches Handinstrument mit dem medizinischen Motorhandstück offenbarungsgemäß durch den Gegenstand des Anspruchs 10 gelöst. Vorteilhafte Ausführungsformen sind in den Unteransprüchen beansprucht.

Konkret wird die Aufgabe gelöst durch ein medizinisches Motorhandstück zum Antreiben eines distalen Endeffektors, mit einem Handgriffabschnitt, einem vorzugsweise hülsenförmigen Bedienelement und einer Rast- und/oder Anschlageinheit. Das hülsenförmige Bedienelement ist um eine Handgrifflängsachse drehbar an einem distalen Endabschnitt des Handgriffabschnitts gehalten und mit dem Endeffektor derart koppelbar, um eine Drehbewegung des Bedienelements in eine Bewegung des Endeffektors zu transformieren oder eine Funktion am Endeffektor zu bewirken. Die Rast- und/oder Anschlageinheit ist dafür vorgesehen und ausgebildet, die Drehbewegung des Bedienelements an Endpositionen zu begrenzen und/oder einen Drehbewegungswiderstand auf das Bedienelement in wenigstens einer Drehzwischenposition zu erhöhen. Die Rast- und/oder Anschlageinheit weist zumindest einen Schraubstift, welcher längs zur Handgrifflängsachse sich erstreckend in ein Einstellgewinde des Bedienelements eingeschraubt ist, und eine Abgleitkulisse auf, die drehfest am Handgriffabschnitt gehalten und mit dem zumindest einen Schraubstift in Gleiteingriff bringbar ist.

In anderen Worten beinhaltet das medizinische Motorhandstück den Handgriffabschnitt, der vorgesehen und ausgebildet ist, von einem Benutzer gehalten zu werden, das Bedienelement, welches vorgesehen und ausgebildet ist, den distalen Endeffektor zu manipulieren, und die Rast- und/oder Anschlageinheit.

Eine Bedienung des Bedienelements erfolgt durch ein Rotieren des vorzugsweise holzylinderförmigen Bedienelements um eine Mittelachse des Bedienelements. Die Rast- und/oder Anschlageinheit begrenzt einen Drehwinkel des Bedienelements um die Mittelachse. Alternativ und/oder zusätzlich erhöht die Rast- und/oder Anschlageinheit den Drehbewegungswiderstand an der zumindest einen Drehzwischenposition dergestalt, dass der Benutzer/Bediener/Verwender ein haptisches Feedback an der zumindest einen Drehzwischenposition bei der Bedienung des Bedienelements erhält.

Die Rast- und/oder Anschlageinheit beinhaltet den zumindest einen Schraubstift, der im Wesentlichen parallel oder alternativ in einem spitzen Winkel zu der Mittelachse des Bedienelements orientiert ist. Der zumindest eine Schraubstift ist in das Einstellgewinde des Bedienelements eingeschraubt, wobei ein Schraubenkopfantrieb des Schraubstifts in distaler Richtung ausgerichtet ist. Die Rast- und/oder Anschlageinheit beinhaltet weiterhin die Abgleitkulisse, welche proximal zu dem Schraubstift angeordnet ist. Die Abgleitkulisse ist drehfest mit dem Handgriffabschnitt verbunden. Ein proximaler Endabschnitt des Schraubstiftes kontaktiert die Abgleitkulisse oder ist zumindest ausgebildet, die Abgleitkulisse zu kontaktieren.

Durch die Ausbildung der Rast- und/oder Anschlageinheit mit dem zumindest einen Schraubstift und dem Einstellgewinde, in welches der zumindest eine Schraubstift eingreift, kann eine räumliche Anordnung zwischen Schraubstift und Abgleitkulisse optimal eingestellt werden. In anderen Worten kann eine exakte Positionierung der Rast- und/oder Anschlageinheit in Axialrichtung des medizinischen Motorhandstücks erfolgen und auf diese Weise eine präzise Anschlagposition sichergestellt werden. Toleranzen der verschiedenen Bauteile zueinander können durch die beschriebene Offenbarung und die sich hieraus ergebende stufenlose Justierbarkeit der Rast-und/oder Anschlageinheit vollständig eliminiert werden. Ein Haptisches Feedback für den Benutzer kann optimal eingestellt werden und damit eine (blinde) Bedienbarkeit und die Patientensicherheit erhöht werden.

Kern der Erfindung ist das medizinische Motorhandstück mit der Rast- und/oder Anschlageinheit, wobei der zumindest eine Schraubstift der Rast- und/oder Anschlageinheit in ein Einstellgewinde, welches sich längs zu dem medizinischen Motorhandstück erstreckt, eingeschraubt ist und die Rast- und/oder Anschlageinheit genau justiert werden kann.

In einem ersten Aspekt kann es sich bei dem zumindest einen Schraubstift um eine Mehrzahl von Schraubstiften handeln, welche als Anschlagstift und/oder als Kugeldruckstück ausgebildet sein können, wobei eine Anzahl an Anschlagstiften vorzugsweise einer Anzahl an Kugeldruckstücken entspricht.

In anderen Worten kann der zumindest eine Schraubstift als der Anschlagstift und/oder als das Kugeldruckstück ausgebildet sein. Vorzugsweise ist sowohl zumindest ein Anschlagstift als auch zumindest ein Kugeldruckstück ausgebildet. Der Anschlagstift kann vorgesehen und ausgebildet sein, den Drehwinkel des Bedienelements um die Mittelachse des Bedienelements zu begrenzen. Das Kugeldruckstück kann vorgesehen und ausgebildet sein, den Drehbewegungswiderstand an der zumindest einen Drehzwischenposition zu erhöhen. Das Kugeldruckstück kann an einem proximalen Endabschnitt des Kugeldruckstücks eine durch eine Feder federvorgespannte Kugel beinhalten, welche an der zumindest einen Drehzwischenposition in eine kalottenförmige Vertiefung der Abgleitkulisse eingreift. Um das Bedienelement aus der Drehzwischenposition zu bewegen, muss zumindest eine Federkraft der Feder überwunden werden, was ein haptisches Feedback für den Benutzer bedeutet.

Durch ein Ausbilden des zumindest einen Schraubstifts als Anschlagstift und/oder als Kugeldruckstück kann sowohl ein maximaler Verdrehwinkel des Bedienelements definiert/begrenzt sein als auch die zumindest eine Drehzwischenposition für den Benutzer haptisch erfühlbar gemacht werden.

In einem weiteren Aspekt kann das Kugeldrückstück eine federvorgespannte Kugel beinhalten, wobei eine Vorspannkraft des Kugeldrückstücks über das Einstellgewinde einstellbar sein kann.

In einem weiteren Aspekt kann es sich bei dem zumindest einen Schraubstift um genau zwei Schraubstifte handeln, welche auf einem konstanten Radius um eine Mittelfaster des Motorhandstücks, vorzugsweise gegenüberliegend auf einem Kreisdurchmesser, angeordnet sein können.

In anderen Worten kann die Rast- und/oder Anschlageinheit jeweils genau den einen Anschlagstift und das eine Kugeldruckstück beinhalten. Der Anschlagstift und das Kugeldruckstück können diametral gegenüberliegend bezogen auf eine Mittelachse des Bedienelements angeordnet sein.

Durch eine derartige Anordnung der Schraubstifte kann eine gleichmäßige axiale Krafteinleitung der Schraubstifte in die Abgleitkulisse sichergestellt werden und ein Verkippen der Abgleitkulisse, was zu einer Verklemmung des Bedienelements führen könnte, verhindert werden.

In einem weiteren Aspekt kann sich an das Einstellgewinde in distaler Richtung eine Einstellbohrung anschließen.

Anders ausgedrückt kann das Einstellgewinde in distaler Richtung durch die Einstellbohrung fortgesetzt sein. Dabei kann sich die Einstellbohrung in einer identischen Orientierung wie das Einstellgewinde längs des medizinischen Motorhandstücks erstrecken.

Alternativ kann eine Erstreckungsrichtung der Einstellbohrung in einem Winkel, vorzugsweise kleiner 10 °, zu einer Erstreckungsrichtung des Einstellgewindes geneigt sein.

Die Einstellbohrung kann sich zwischen einem distalen Endabschnitt des Einstellgewindes und einem distalen Endabschnitt des Bedienelements, vorzugsweise einer distalen Endfläche des Bedienelements, erstrecken.

Durch die Einstellbohrung kann der zumindest eine Schraubstift in einem montierten Zustand justiert werden. In anderen Worten kann die Rast- und/oder Anschlageinheit in einem Zustand, in welchem das medizinische Motorhandstück vollständig oder zumindest beinahe vollständig montiert ist (fein-) justiert werden.

In einem weiteren Aspekt kann die Einstellbohrung in distaler Richtung zu einer Mittelfaser des medizinischen Motorhandstücks hin geneigt orientiert sein.

In anderen Worten kann ein Abstand zwischen einer Mittelfaser der Einstellbohrung und der Mittelfaser des medizinischen Motorhandstücks in einem distalen Abschnitt der Einstellbohrung kleiner sein als in einem proximalen Abschnitt der Einstellbohrung.

Durch eine Neigung der Einstellbohrung kann eine Außengeometrie des medizinischen Motorhandstücks derart gestaltet sein, dass das medizinische Motorhandstück sich in distaler Richtung verschlankt. Auf diese Weise kann eine Handhabbarkeit des medizinischen Motorhandstücks verbessert werden.

In einem weiteren Aspekt kann die Einstellbohrung distal durch einen Rastring verschlossen sein.

In anderen Worten kann das Bedienelement distal durch den Rastring begrenzt sein.

Auf diese Weise kann verhindert werden, dass die Justierung des zumindest einen Schraubstifts durch einen unautorisierten Dritten verändert wird oder es zu einer unbeabsichtigten Veränderung der Justierung des zumindest einen Schraubstifts kommt. Weiterhin verhindert der Rastring ein Eindringen von Schmutz und Fremdpartikeln in die Einstellbohrung. Bevorzugt ist der Rastring drehfest mit dem Hangriffabschnitt verbunden. Weiterhin bevorzugt ist der Rastring derart mit dem medizinischen Motorhandstück verbunden, dass der Rasrting nicht werkzeuglos demontiert werden kann.

In einem weiteren Aspekt kann der zumindest eine Schraubstift mittels Klebstoff fixiert sein.

Anders ausgedrückt kann nach einer Justierung des zumindest einen Schraubstifts ein Klebstoff in die Einstellbohrung eingebracht werden und/oder die Einstellbohrung mit Klebstoff verfüllt werden.

Auf diese Weise kann verhindert werden, dass die Justierung des zumindest einen Schraubstifts durch einen unautorisierten Dritten verändert wird oder es zu einer unbeabsichtigten Veränderung der Justierung des zumindest einen Schraubstifts kommt. Weiterhin verhindert der Klebstoff ein Eindringen von Schmutz und Fremdpartikeln in die Einstellbohrung.

In einem weiteren Aspekt kann die Abgleitkulisse ringförmig ausgebildet sein und auf einer distalen Stirnseite einen ersten Abschnitt mit Rastlöchern und einen zweiten glatten Abschnitt beinhalten.

In anderen Worten kann die Abgleitkulisse den ersten Abschnitt beinhalten, der vorgesehen und ausgebildet ist, mit dem vorzugsweise einen Anschlagstift in Gleitkontakt zu stehen und den zweiten Abschnitt beinhalten, der Rastlöcher bzw. kalottenförmige Vertiefungen beinhaltet, welche vorgesehen und ausgebildet sind, mit dem vorzugsweise einen Kugeldruckstück in Kontakt zu stehen.

Durch die Segmentierung der Abgleitkulisse kann sowohl eine Rastfunktion als auch eine Anschlagfunktion der Rast- und/oder Anschlageinheit zuverlässig umgesetzt werden. In anderen Worten kann durch eine Funktionstrennung eine Ausfallsicherheit der Rast- und/oder Anschlageinheit erhöht werden.

In einem weiteren Aspekt können der erste Abschnitt und der zweite Abschnitt durch Anschläge getrennt sein, welche in distaler Richtung von der Abgleitkulisse hervorstehen.

In einem weiteren Aspekt kann der Schraubenkopfantrieb des zumindest einen Schraubstifts als Schlitzschraubenkopf oder als Innensechskantkopf ausgebildet sein. Es sind jedoch weitere geeignete Schraubenkopfantriebe vorstellbar.

In einem weiteren Aspekt kann eine Federkraft des Kugeldrückstücks durch eine Einstellschraube einstellbar sein.

Bei einem Justierungsvorgang können der zumindest eine Schraubstift mit einem vordefinierten Drehmoment angezogen werden und damit eine definierte Kontaktkraft zwischen Schraubstift und Abgleitkulisse gewährleistet werden.

Die Aufgabe der vorliegenden Offenbarung wird weiterhin gelöst durch ein medizinisches Handinstrument, welches ein medizinisches Motorhandstück nach einem der obigen Aspekte, einen Werkzeugschaft und einen Effektor beinhaltet.

In einem Aspekt kann der Endeffektor relativ zu dem Werkzeugschaft abwinkelbar sein.

### Kurzbeschreibung der Figuren

Die Offenbarung wird nachfolgend anhand bevorzugter Ausführungsbeispiele mit Hilfe von Figuren näher erläutert. Es zeigen:
- Fig. 1: eine perspektivische Ansicht eines medizinischen Handinstruments;
- Fig. 2: eine weitere perspektivische Ansicht des medizinischen Handinstruments;
- Fig. 3: eine Teillängsschnittansicht eines Motorhandstücks des medizinischen Handinstruments;
- Fig. 4: eine Querschnittansicht des Motorhandstücks des medizinischen Handinstruments;
- Fig. 5: eine Teillängsschnittansicht eines distalen Endabschnitts des medizinischen Handinstruments in einer geraden Schaftform;
- Fig. 6: eine Teillängsschnittansicht des distalen Endabschnitts des medizinischen Handinstruments in einer abgewinkelten Schaftform;
- Fig. 7: eine Teillängsschnittansicht des distalen Endabschnitts des medizinischen Handinstruments in einem Freigabezustand;
- Fig. 8: eine isometrische Perspektivansicht des Motorhandstücks des medizinischen Handinstruments;
- Fig. 9: eine Teillängsschnittansicht des Motorhandstücks des medizinischen Handinstruments;
- Fig. 10: eine perspektivische Längsschnittansicht des medizinischen Handinstruments gemäß einer Modifikation;
- Fig. 11: eine Detailansicht einer Abgleitkulisse des medizinischen Handinstruments gemäß der Modifikation;
- Fig. 12: eine weitere Teillängsschnittansicht des Motorhandstücks des medizinischen Handinstruments;
- Fig. 13: eine weitere Teillängsschnittansicht des Motorhandstücks des medizinischen Handinstruments;
- Fig. 14: eine perspektivische Teilansicht des Motorhandstücks;
- Fig. 15: eine Frontansicht des Motorhandstücks;
- Fig. 16: eine Detailansicht eines Anschlagstifts;
- Fig. 17: eine Detailansicht eines Kugeldrückstücks.

Die Figuren sind schematischer Natur und dienen lediglich dem Verständnis der Offenbarung. Gleiche Elemente sind mit denselben Bezugszeichen versehen. Die Merkmale der verschiedenen Ausführungsbeispiele können untereinander ausgetauscht werden.

### Detaillierte Beschreibung bevorzugter Ausführungsformen

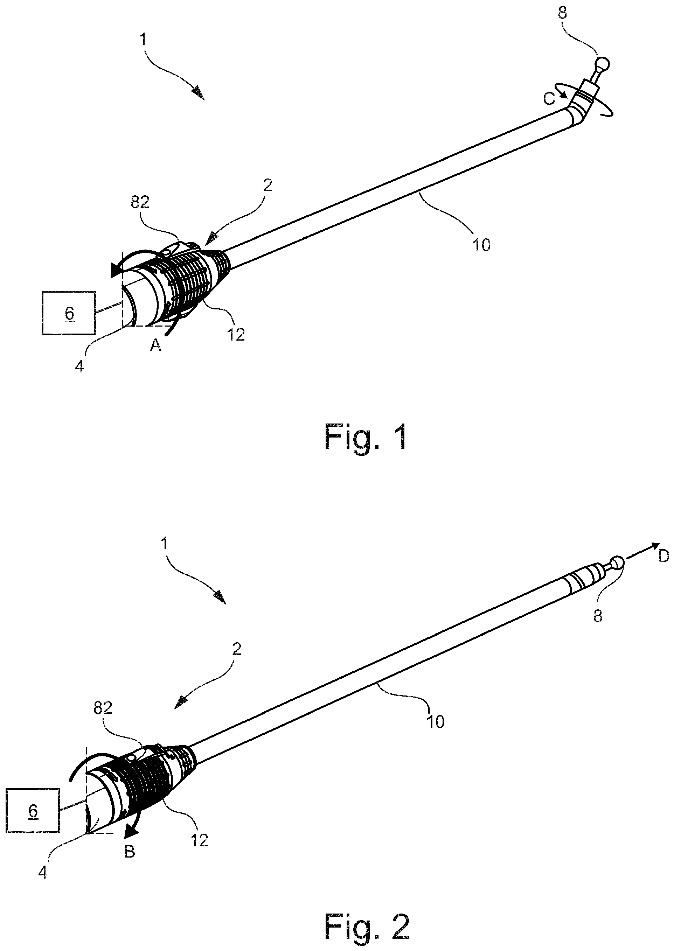

Figur 1 zeigt perspektivisch ein medizinisches Handinstrument 1 gemäß einer Ausführungsform der vorliegenden Offenbarung. Das medizinische Handinstrument 1 weist dabei ein offenbarungsgemäßes medizinisches Motorhandstück 2 auf, welches einen proximalen Handgriffabschnitt 4 aufweist und welches, wie in Fig. 1 schematisch dargestellt, mit einer Antriebseinheit 6 gekoppelt werden kann. Alternativ kann die Antriebseinheit 6 auch in dem Motorhandstück 2 aufgenommen sein und über einen Energieversorgungsanschluss mit Energie versorgt werden.

Ferner weist das Handinstrument 1 ein distales Werkzeug (Endeffektor/Effektorabschnitt) 8 auf, welches über einen (Werkzeug-)Schaft 10 mit dem Motorhandstück 2, insbesondere mit dem Handgriffabschnitt 4, gekoppelt werden kann oder von diesem entkoppelt werden kann. Das Werkzeug 8 kann dabei beispielsweise als Fräser, Bohrer oder Polierkopf ausgeführt sein.

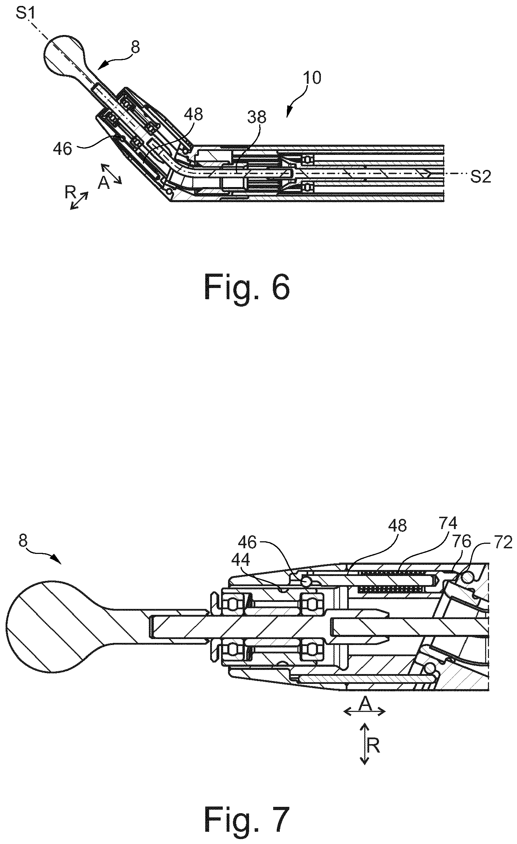

Wenn der Schaft 10 mit dem Handgriffabschnitt 4 gekoppelt ist, wird Drehmoment von der Antriebseinheit 6 über einen in dem Handinstrument 1, insbesondere dem Handgriffabschnitt 4 und dem Schaft 10, angeordneten Drehmoment-Übertragungszug hin zu dem Werkzeug 8 übertragen, um dieses in Drehung zu versetzen. Bei Verwendung des Handinstruments 1 im Rahmen einer chirurgischen Operation bzw. medizinischen Behandlung ist es bekannt, dass das Werkzeug 8 relativ zu dem Schaft 10 abgewinkelt werden kann (in Fig. 1 durch den Pfeil C angedeutet). D.h. das Werkzeug 8 und der Schaft 10 können, wie nachstehend näher beschrieben, so zueinander abgewinkelt werden, dass eine Längsachse S1 des Werkzeugs 8 und eine Schaftlängsachse S2 des Schafts 10 miteinander einen Winkel ungleich 180°, insbesondere kleiner als 180°, ausbilden (vgl. Fig. 6).

Wie in Fig. 1 gezeigt, ist hierfür bei dem offenbarungsgemäßen Handinstrument 1 ein hülsenförmiges Bedienelement 12 vorgesehen. Das Bedienelement 12 ist dabei an einem distalen Endabschnitt des Handgriffabschnitts 4 angeordnet und um eine Handgrifflängsachse in einer ersten Drehrichtung A und in einer der ersten Drehrichtung A entgegengesetzten, zweiten Drehrichtung B drehbar. Mit anderen Worten ausgedrückt, weist das Motorhandstück 2 den Handgriffabschnitt 4 und das distal angeordnete Bedienelement 12 auf, welche koaxial zueinander angeordnet sind, wobei das Bedienelement 12 relativ zu dem Handgriffabschnitt 4 gedreht werden kann.

Eine Drehung des Bedienelements 12 aus einer neutralen Nullstellung, bei welcher das Werkzeug 8 nicht relativ zu dem Schaft 10 abgewinkelt ist, in der ersten Drehrichtung A bewirkt dabei, wie in Fig. 1 gezeigt, ein Abwinkeln des Werkzeugs 8 relativ zu dem Schaft 10. Die erste Drehrichtung A ist bei dem Handinstrument 1 gemäß der vorliegenden Offenbarung als eine Drehung des Bedienelements 12 relativ zu dem Handgriffabschnitt 4 nach links definiert. Anders ausgedrückt entspricht die erste Drehrichtung A in einer distalen Draufsicht auf das Werkzeug 8 einer Drehung des Bedienelements 12 im Uhrzeigersinn.

Wie in Fig. 2 gezeigt, kann das Bedienelement 12 bei dem offenbarungsgemäßen Handinstrument 1 auch in der zweiten Drehrichtung B, welche der ersten Drehrichtung A entgegengesetzt ist, relativ zu dem Handgriffabschnitt 4 gedreht werden. D.h. die zweite Drehrichtung B ist in der Draufsicht auf das Werkzeug 8 als eine Drehung des Bedienelements 12 entgegen dem Uhrzeigersinn bzw. als eine Drehung nach rechts definiert. Wie durch den Pfeil D in Fig. 2 angedeutet, bewirkt eine Drehung des Bedienelements 12 in der zweiten Drehrichtung B ein Lösen der Kopplung des Werkzeugs 8 mit dem Schaft 10. Somit kann, wie nachstehend näher erläutert, durch eine Drehung des Bedienelements 12 in der zweiten Drehrichtung C ein einfacher und schneller Werkzeugwechsel gewährleistet werden.

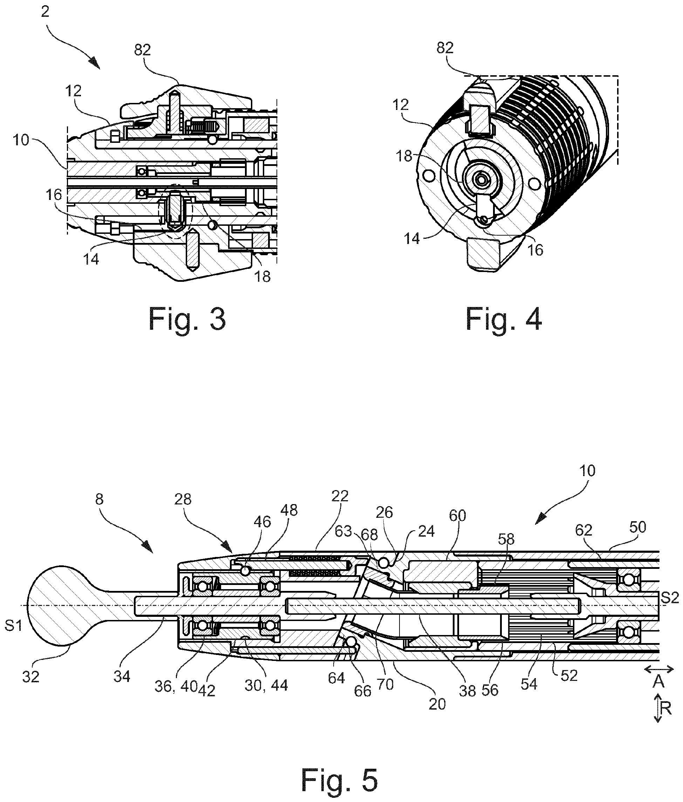

Fign. 3 und 4 zeigen Teilschnittansichten des Handinstruments 1 gemäß der ersten Ausführungsform. Zur Übertragung der Drehbewegung des Bedienelements 12 auf den Schaft 10 ist in radialer Richtung des Bedienelements 12 ein Verstellstift 14 angeordnet. Ein radial außenliegender Endabschnitt des Verstellstifts 14 ist dabei in einer Axialnut 16, welche auf einer Innenumfangsfläche des Bedienelements 12 ausgebildet ist, aufgenommen. Auf einer radialen Innenseite ist der Verstellstift 14 in einer Drehübertragungshülse 18 aufgenommen. Die Drehübertragungshülse 18 ist dabei drehfest mit dem Schaft 10 verbunden, so dass eine Bewegung des Verstellstifts 14 in Umfangsrichtung eine Drehung der Drehübertragungshülse 18 und des Schafts 10 bewirkt. Anders ausgedrückt, bewegt sich bei Verdrehung des Bedienelements 12 in der ersten Drehrichtung A oder in der zweiten Drehrichtung B der Verstellstift 14 in der Axialnut 16 mit dem Bedienelement 12 mit und bewirkt je nach Drehrichtung das Abwinkeln bzw. den Auswurf des Werkzeugs 8.

Fig. 5 zeigt einen Längsquerschnitt eines distalen Endabschnitts des Handinstruments 1 in einer geraden Schaftform. D.h. das Bedienelement 12 ist nicht relativ zu dem Handgriffabschnitt 4 verdreht (Nullstellung). Demzufolge sind das Werkzeug 8 und der Schaft 10 nicht abgewinkelt und die Längsachse S1 des Werkzeugs 8 ist kollinear zu der Schaftlängsachse S2. Wie in Fig. 5 zu erkennen, weist der Schaft 10 einen, dem Handgriffabschnitt 4 zugewandten und mit diesem koppelbaren, proximalen Schaftabschnitt 20 und einen mit dem Werkzeug 8 koppelbaren distalen Schaftabschnitt 22 auf. Wenn das Bedienelement 12 nicht relativ zu dem Handgriffabschnitt 4 verdreht ist und der Schaft 10 in der geraden Schaftform vorliegt, sind der proximale Schaftabschnitt 20 und der distale Schaftabschnitt 22 demzufolge in einer Linie angeordnet bzw. weisen einen Winkel von 0° zueinander auf. Der proximale Schaftabschnitt 20 ist dabei im Wesentlichen rohrförmig ausgestaltet und weist an seinem distalen Endabschnitt eine in Schaftlängsachse S2 angestellte Stirnseite 24 auf. Der distale Schaftabschnitt 22 ist ebenfalls annähernd rohrförmig ausgeführt. Das Rohr läuft zum distalen Ende hin spitzförmig zu. Der distale Schaftabschnitt 22 weist an seinem proximalen Endabschnitt eine in Schaftlängsachse S2 angestellte Stirnseite 26 auf.

Die angestellten Stirnseiten 24, 26 weisen jeweils einen Anstellwinkel von vorzugsweise 22,5° zu einer Normalenebene zur Schaftlängsachse S2 auf. Wenn der Schaft 10 in einer geraden Schaftform bzw. ausgestreckt ist, sind die beiden angestellten Stirnseiten 24, 26 derart zueinander versetzt, dass sich die langen Enden der angestellten Stirnseiten 24, 26 in Relation zur Schaftlängsachse S2 gegenüberliegen. Die angestellten Stirnseiten 24, 26 liegen aufeinander auf. Die angestellten Stirnseiten 24, 26 müssen dabei nicht zwangsläufig einen Anstellwinkel von 22,5° aufweisen. Es sind auch Anstellwinkel von beispielsweise 10°, 18°, 30°, 45° oder jeder andere Anstellwinkel denkbar.

Wie vorstehend erwähnt, ist der distale Schaftabschnitt 22 ausgebildet, mit dem Werkzeug 8 gekoppelt zu werden. D.h. der distale Schaftabschnitt 22 stellt eine Werkzeugaufnahme dar, wobei das Werkzeug 8 in der Werkzeugaufnahme aufgenommen ist und relativ zu der Werkzeugaufnahme, d.h. dem distalen Schaftabschnitt 22, drehbar gelagert ist.

Hierfür ist in dem distalen Schaftabschnitt 22 eine erste Kupplungsvorrichtung 28 angeordnet. An dem Werkzeug 8 ist eine zweite Kupplungsvorrichtung 30 vorgesehen, die in einem Kupplungszustand durch Zusammenwirken mit der ersten Kupplungsvorrichtung 28 eine Kupplung zwischen dem Werkzeug 8 und dem distalen Schaftabschnitt 22 realisiert, um das Werkzeug 8 im distalen Schaftabschnitt 22 in Axialrichtung A zu fixieren.

Wie vorstehend erwähnt, schließen die Längsachse S1 des Werkzeugs 8 bzw. des distalen Schaftabschnitts 22 und die Schaftlängsachse S2, d.h. die Längsachse des proximalen Schaftabschnitts 20 in Fig. 5 einen Winkel von 0° ein. Zudem ist in Fig. 5 ein Kupplungszustand zwischen dem Werkzeug 8 und dem distalen Schaftabschnitt 22 gezeigt. Das heißt, dass die erste Kupplungsvorrichtung 28, die im/am distalen Schaftabschnitt 22 angebracht ist, und die zweite Kupplungsvorrichtung 30, die am Werkzeug 8 vorgesehen ist, miteinander in Eingriff stehen bzw. zusammenwirken. Das Werkzeug 8 weist einen Werkzeugkopf/Effektor 32, beispielsweise einen Fräskopf, und einen Werkzeugschaft 34 auf. Der Werkzeugkopf 32 und der Werkzeugschaft 34 sind drehfest miteinander verbunden.

In Fig. 5 ist weiterhin zu erkennen, dass der Werkzeugschaft 34 mithilfe einer Wälzlagereinheit 36 in dem distalen Schaftabschnitt 22 drehbar gelagert ist. Durch den proximalen Schaftabschnitt 20 erstreckt sich eine Antriebswelle 38, welche drehfest mit dem Werkzeugschaft 34 verbunden ist und in Form eines Drehmomentübertragungszugs Drehmoment von der Antriebseinheit 6 auf den Werkzeugschaft 34 aufbringt. Der Werkzeugschaft 34 dreht sich infolge dieser Drehmomentbeaufschlagung relativ zu dem distalen Schaftabschnitt 22. Die Wälzlagereinheit 36 weist zumindest ein, hier genau zwei, voneinander in Axialrichtung A beabstandete Wälzlager 40, genauer gesagt Kugellager, auf, die alternativ aber auch als Gleitlager ausgebildet sein können. Die Wälzlager 40 sind in einem Lagergehäuse 42, das Teil der Wälzlagereinheit 36 ist, aufgenommen.

Die zweite Kupplungsvorrichtung 30 ist in das Lagergehäuse 42 eingebracht und damit nicht direkt an dem Werkzeug 8 vorgesehen. Die Wälzlagereinheit 36 und damit auch das Lagergehäuse 42 ist an dem Werkzeugschaft 34 in Axialrichtung A fixiert angeordnet. Alternativ dazu wäre es aber auch denkbar, dass die zweite Kupplungsvorrichtung 30 direkt an dem Werkzeugschaft 34 vorgesehen ist. Die zweite Kupplungsvorrichtung 30 ist als eine sich in Umfangsrichtung des Lagergehäuses 42 durchgängig ersteckende bzw. umlaufende Axialsicherungsnut 44 ausgebildet. Die Axialsicherungsnut 44 hat vorzugsweise einen halbkreisförmigen oder kreissegmentförmigen Querschnitt.

Die erste Kupplungsvorrichtung 28, die in dem distalen Schaftabschnitt 22 vorgesehen ist, weist zumindest eine Verriegelungskugel 46 auf. Der Durchmesser der Verriegelungskugel 46 ist so gewählt, dass die Verriegelungskugel 46 in der Axialsicherungsnut 44 aufnehmbar ist. Vorzugsweise umgibt die Axialsicherungsnut 44 zumindest einen die Axialsicherungsnut 44 kontaktierenden Abschnitt der Verriegelungskugel 46 vollumfänglich. Die Verriegelungskugel 46 wird im Kupplungszustand an ihrer der Axialsicherungsnut 44 gegenüberliegenden Seite von einem distalen Ende eines Raststiftes/Schiebers 48 in der Axialsicherungsnut 44 gehalten. Der Raststift 48 ist ein Teil der ersten Kupplungsvorrichtung 28. Der Raststift 48 ist in Radialrichtung R fixiert. Der Raststift 48 ist in Axialrichtung verschieblich bzw. beweglich in dem distalen Schaftabschnitt 22 angeordnet.

In dem in Fig. 5 gezeigten Kupplungszustand ist also die Verriegelungskugel 46 in der Axialsicherungsnut 44 aufgenommen und wird von dem Raststift 48 in Radialrichtung in der Axialsicherungsnut 44 gehalten. Diese Position des Raststiftes 48 in Axialrichtung A wird als Kupplungsposition bezeichnet. Eine Kante 63 am distalen Ende des proximalen Schaftabschnittes 20 verhindert eine Bewegung des Raststiftes 48 in Axialrichtung hin zu dem proximalen Schaftabschnitt 20.

Der proximale Schaftabschnitt 20 weist ein feststehendes Außenrohr 50, ein Hohlrad 52 mit einer Innenverzahnung 54, ein Ritzel 56 mit einer Außenverzahnung 58 und eine exzentrische Sicherungsbuchse 60 auf. Das Hohlrad 52 ist innerhalb des Außenrohrs 50 positioniert und die Längsachse des Außenrohrs 50 entspricht der Längsachse des Hohlrads 52. Das Außenrohr 50 und das Hohlrad 52 sind also konzentrisch angeordnet. Das Hohlrad 52 ist über eine in dem proximalen Schaftabschnitt 20 aufgenommene Hohlwelle 62 mit der Drehübertragungshülse 18, d.h. mit dem Bedienelement 12, verbunden.

Wie vorstehend erwähnt, ist das Außenrohr 50 als feststehendes Rohr ausgeführt und bewegt sich demnach nicht. Das distale Ende des Außenrohrs 50 weist die angestellte Stirnseite 24 auf. Das distale Ende des Außenrohrs 50 weist weiterhin eine Aufnahmebohrung 64 und einen Aufnahmezapfen für ein Wälzlager 66 auf. Das Außenrohr 50 weist eine Rille/ Nut für die Kugeln des Wälzlagers 66 auf. Auf dem Wälzlager 66 ist der distale Schaftabschnitt 22 gelagert.

Die Innenverzahnung 54 kämmt mit der Außenverzahnung 58 des Ritzels 56. Dadurch wird eine Rotation des Hohlrads 52, die durch das Bedienelement 12 gesteuert wird, auf das Ritzel 56 übertragen. Die Drehrichtung des Ritzels 56 ist dabei gleichgesetzt der Drehrichtung des Hohlrads 52. Das Ritzel 56 wird vom Hohlrad 52 angetrieben, das Ritzel 56 dreht sich jedoch in der exzentrischen Sicherungsbuchse 60. Die Sicherungsbuchse 60 ist exzentrisch zum Hohlrad 52 angeordnet. D.h. die Längsachse der exzentrischen Sicherungsbuchse 60 ist zwar parallel zur Längsachse des Hohlrads 52, die Längsachsen liegen aber nicht übereinander bzw. versetzt zueinander. Der distale Schaftabschnitt 22 weist eine Verstellbuchse 68 auf. Die Verstellbuchse 68 ist in der Aufnahmebohrung 64 des proximalen Schaftabschnitts 20 gelagert. Die Verstellbuchse 68 ist über einen flexiblen Silikonschlauch 70 mit dem Ritzel 56 derart verbunden, dass eine Rotation des Ritzels 56 auf die Verstellbuchse 68 übertragen wird. Hierfür ist der flexible Silikonschlauch 70 an der Verstellbuchse 68 und dem Ritzel 56 beispielsweise durch Schweißen oder Kleben befestigt. Die Verstellbuchse 68 ist ferner über einen Mitnahmezapfen (nicht dargestellt) mit dem distalen Schaftabschnitt 22 formschlüssig verbunden, so dass eine Rotation der Verstellbuchse 68 auf den distalen Schaftabschnitt 22 übertragen wird.

Fig. 6 zeigt einen Längsquerschnitt durch den distalen Endabschnitt des Schafts 10, wobei der distale Schaftabschnitt 22 zu dem proximalen Schaftabschnitt 20 bei einem jeweiligen Anstellwinkel der beiden Stirnseiten 24, 26 um 22,5° folglich um 45° abgewinkelt ist. Der distale Schaftabschnitt 22 ist im Vergleich zur Stellung in Fig. 5 um 180° um die eigene Längsachse S1 gedreht. Die angestellten Stirnseiten 24, 26 liegen in dieser Stellung wieder komplett / flächig aufeinander auf. Durch die Drehung des distalen Schaftabschnitts 22 sind die langen Enden der angestellten Stirnseiten 24, 26 aber nebeneinander positioniert. Die Anstellwinkel der angestellten Stirnseiten 24, 26 addieren sich somit. Dadurch wird der distale Schaftabschnitt 22 im Vergleich zum proximalen Schaftabschnitt 20 um den doppelten Anstellwinkel der angestellten Stirnseiten 24, 26 abgewinkelt.

In der in Fig. 6 gezeigten Stellung, ist der Mitnahmezapfen gegenüber der Sicherungsbuchse 60 angeordnet. D.h. die Verstellbuchse 68 hat sich von der ausgestreckten Position zur maximalen Abwinklung um 180° gedreht. Die 45°-Position stellt für diese Konstruktion den Umkehrpunkt dar. Die Verstellbuchse 68 hat sich in dieser Position um 180° gedreht. Bei weiterer Drehung des Hohlrads 52, d.h. des Bedienelements 12, würde sich der distale Schaftabschnitt 22 wieder in die Ausgangsstellung (Nullstellung) zurück drehen.

Ferner ist in Fig. 6 zu erkennen, dass die Verriegelungskugel 46 auch dann, wenn der distale Schaftabschnitt 22 in der maximal einstellbaren Winkelstellung bzw. maximalen Abwinklung bezüglich des proximalen Schaftabschnitts 20 abgewinkelt ist, durch den Raststift 48 in Radialrichtung R in der Axialsicherungsnut 44 gehalten ist. Auf diese Weise ist das Werkzeug 8 in dem distalen Schaftabschnitt durch den Eingriff zwischen erster Kupplungsvorrichtung 28 und zweiter Kupplungsvorrichtung 30 auch in dieser Winkelstellung in Axialrichtung A gesichert.

Der Drehmomentstrang, insbesondere die Antriebswelle 38, hin zum Werkzeugschaft 34, der im Übergangsbereich zwischen dem distalen Schaftabschnitt 22 und dem proximalen Schaftabschnitt 20 angeordnet ist, ist flexibel. Dieser flexible Abschnitt des Drehmomentstrangs erlaubt, dass der distale Abschnitt des Werkzeugschaftes 34 mit Werkzeugkopf 32 zusammen mit dem distalen Schaftabschnitt 22 relativ zum Drehmomentstrang im proximalen Schaftabschnitt 20 abgewinkelt werden kann. Gleichzeitig ist der flexible Abschnitt des Drehmomentstrangs so ausgeführt, dass er ein Drehmoment, das auf einen proximalen Abschnitt des Werkzeugschaftes 34 ausgeübt wird, weiterhin auf den Werkzeugkopf 32 übertragen kann.

Fig. 7 ist eine Längsschnittansicht des distalen Endabschnitts des Schafts 10 in einem Freigabezustand zwischen dem Werkzeug 8 und dem distalen Schaftabschnitt 22. D.h. in der in Fig. 7 gezeigten Stellung stehen die die erste Kupplungsvorrichtung 28 und die zweite Kupplungsvorrichtung 30 nicht in Wirkeingriff miteinander, so dass das Werkzeug 8 entnommen bzw. gewechselt werden kann. Um den Wirkeingriff der ersten Kupplungsvorrichtung 28 und der zweiten Kupplungsvorrichtung 30 zu lösen, wird das Bedienelement 12 in der zweiten Drehrichtung B gedreht (vgl. Fig. 2). Wie nachstehend näher erläutert, ist die Verriegelungskugel 46 dabei nicht mehr in der Axialsicherungsnut 44 aufgenommen. Stattdessen ist sie von dem Raststift 48 in Radialrichtung R gegen eine Außenumfangsfläche des Lagergehäuses 42 gehalten.

Damit sich die Verriegelungskugel 46 ausgehend von dem, in Fig. 5 gezeigten Kupplungszustand, aus der Axialsicherungsnut 44 heraus in den Freigabezustand bewegen kann, muss der Raststift 48 sich in Axialrichtung A hin zu dem proximalen Schaftabschnitt 20 bewegen. Dies wird im Kupplungszustand durch die umlaufende Kante 63 des proximalen Schaftabschnitts 20 verhindert. Die Kante 63 ist jedoch an einer Stelle einer Raststift-Aufnahmevertiefung 72 unterbrochen. Wenn das Bedienelement 12 in der zweiten Drehrichtung B gedreht wird, rotiert der distale Schaftabschnitt 22 relativ zu dem proximalen Schaftabschnitt 20 in einer dem Abwinkeln entgegengesetzten Richtung, beispielsweise um (-)18°, bis der Raststift 48 relativ zu dem proximalen Schaftabschnitt 20 so positioniert ist, dass er in Umfangsrichtung auf einer Höhe mit der Raststift-Aufnahmevertiefung 72 ist. Ein Vorspannelement 74, das ein Teil der ersten Kupplungsvorrichtung 28 ist, drückt den Raststift 48 in Axialrichtung A zum proximalen Schaftabschnitt 20 hin. Somit schiebt das Vorspannelement 74 den Raststift 48, genauer gesagt dessen proximales Ende, das als Rastvorsprung 76 ausgebildet ist, in die Raststift-Aufnahmevertiefung 72 hinein. Die Position, in der der Raststift 48 ist, wenn sein Rastvorsprung 76 in die Raststift-Aufnahmevertiefung 72 eingreift, wird als der Freigabezustand bzw. Freigabeposition bezeichnet.

In der Freigabeposition liegt das distale Ende des Raststifts 48 nicht mehr der Axialsicherungsnut 44 gegenüber. Damit wird die Verriegelungskugel 46 in Radialrichtung R nicht in der Axialsicherungsnut 44 gehalten. Das Werkzeug 8 ist somit relativ zum distalen Schaftabschnitt 22 nicht mehr in Axialrichtung A fixiert. Wird nun, ausgehend vom Kupplungszustand, eine Zugkraft (in Axialrichtung A) auf das distale Ende des Werkzeugs 8 aufgebracht, löst sich die Verriegelungskugel 46 aus der Axialsicherungsnut 44. Auf diese Weise kann das Werkzeug 8 von dem distalen Schaftabschnitt 22 entkuppelt werden.

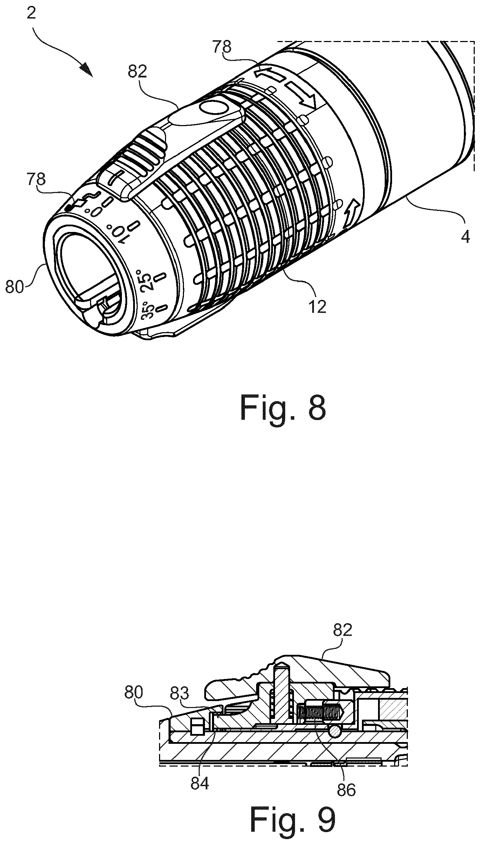

Fig. 8 zeigt eine Perspektivansicht des distalen Endabschnitts des Motorhandstücks 2 gemäß der ersten Ausführungsform, ohne dass der Schaft 10 mit dem Handgriffabschnitt 4 gekoppelt ist. Wie vorstehend beschrieben, bewirkt eine Drehung des Bedienelements 12 in der ersten Drehrichtung A ein Abwinkeln des Werkzeugs 8 und eine Drehung des Bedienelements 12 in der zweiten Drehrichtung B löst die Kupplung zwischen dem Werkzeug 8 und dem Schaft 10, bzw. dem distalen Schaftabschnitt 22, indem der Wirkeingriff zwischen der ersten Kupplungsvorrichtung 28 und der zweiten Kupplungsvorrichtung 30 gelöst wird. Zur Erleichterung der Bedienung sind an dem Bedienelement 12 Indikatoren 78 in Form von Pfeilen, welche die Drehrichtungen angeben, angebracht. In Kombination mit Indikatoren 78 auf einer Indikatorhülse 80, welche distal des Bedienelements 12 drehfest an dem Handgriffabschnitt 4 angebracht ist, kann der Verwender bei der Bedienung des Handinstruments 1 schnell erkennen, welche Drehrichtung welcher Funktion entspricht. Hierfür sind, wie in Fig. 8 gezeigt, auf einer Außenumfangsfläche der Indikatorhülse 80 verschiedene Winkelpositionen, welche jeweils einer definierten Drehung des Bedienelements 12 in der ersten Drehrichtung A und damit einem definierten Abwinkeln des Werkzeugs 8 relativ zu dem Schaft 10 entsprechen, sowie ein Schloss-Symbol, bzw. ein Symbol eines geöffneten Schlosses, aufgebracht. Der Verwender kann somit den einzelnen Drehrichtungen A, B die jeweilige Funktion, nämlich das Abwinkeln oder das Entkuppeln, zuordnen. Das Motorhandstück 2 mitsamt dem Bedienelement 12 ermöglicht somit eine intuitive Bedienung und Integration der beiden Funktionen.

Wie in Fig. 8 gezeigt, ist an dem Motorhandstück 2 gemäß der ersten Ausführungsform ein Verriegelungsschieber 82 angeordnet. Der Verriegelungsschieber 82 ist dabei in Radial- und in Umfangsrichtung fest so in dem Bedienelement 12 aufgenommen, dass sich der Verriegelungsschieber 82 lediglich in Axialrichtung relativ zu dem Bedienelement 12 zwischen einer (distalen) Verriegelungs- und einer (proximalen) Entriegelungsposition bewegen kann. In der Verriegelungsposition kann das Bedienelement 12, wie nachstehend näher erläutert, nicht relativ zu dem Handgriffabschnitt 4 gedreht werden.

Hierfür verrastet in der Verriegelungsposition ein distaler Zapfenabschnitt 83 des Verriegelungsschiebers 82, wie in Fig. 9 gezeigt, mit einem in der Indikatorhülse 80 aufgenommenen Indikatorrastring 84. Der Indikatorrastring 84 weist eine Mehrzahl an über den Umfang verteilten Ausnehmungen auf, in welche der Zapfenabschnitt 83 in der Verriegelungsposition vorragt, um so das Bedienelement 12 in Umfangsrichtung relativ zu dem Handgriffabschnitt 4 festzulegen bzw. festzuhalten. Die Ausnehmungen des Indikatorrastrings 84 entsprechen dabei vorzugsweise den auf der Außenumfangsfläche der Indikatorhülse 80 angebrachten Indikatoren 78. Somit kann der Verwender einfach ein Abwinkeln des Werkzeugs 8 bei einem definierten Winkel einstellen und das abgewinkelte Werkzeug 8 bei diesem Winkel fixieren.

Um das Bedienelement 12 für eine Verstellung des Winkels oder zum Lösen der Kupplung relativ zu dem Handgriffabschnitt 4 drehen zu können, muss der Verriegelungsschieber 82 folglich aus der Verriegelungsposition in die Entriegelungsposition verlagert werden, d.h. in Richtung proximal.

Bei dem Motorhandstück 2 gemäß der hier beschriebenen Ausführungsform ist der Verriegelungsschieber 82 in Axialrichtung über ein Federelement 86 gegen das Bedienelement 12 vorgespannt. Das Federelement 86 ist dabei so zwischen dem Verriegelungsschieber 82 und einer Anschlagsfläche des Bedienelements 12 angeordnet, dass es den Verriegelungsschieber 82 in die Verriegelungsposition, d.h. in Richtung distal, drückt. Das Federelement 86 übt folglich eine automatische Rückstellkraft auf den Verriegelungsschieber 82 aus, um diesen in der Verriegelungsposition zu halten.

Alternativ ist es selbstverständlich auch denkbar, dass das Motorhandstück 2 kein Federelement 86 aufweist. Bei einem solchen Motorhandstück gemäß einer Modifikation der hier beschriebenen Ausführungsform mit manueller Rückstellung muss der Verwender den Verriegelungsschieber 82 zur Entriegelung in Richtung proximal ziehen und zur Verriegelung aktiv in Richtung distal drücken.

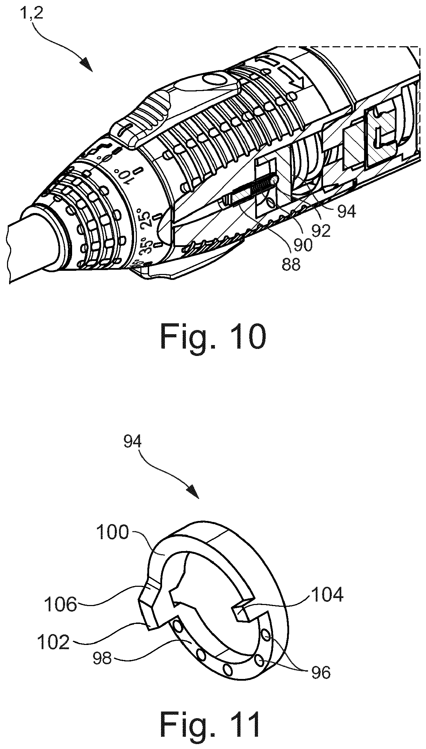

Wie in Fign. 10 und 11 gezeigt, ist bei dem Motorhandstück 2 ein Schraubstift in Form eines Kugeldruckelements 88 vorgesehen. Dieses weist ein Federelement 90 auf, welches eine Kugel 92 in Richtung proximal gegen eine (dreh-) fest in dem Handgriffabschnitt 4 aufgenommene ringförmige Abgleitkulisse 94 (siehe Fig. 11) drückt. Auf der Abgleitkulisse 94 sind kalottenförmige Rastausnehmungen 96 ausgebildet, welche die Kugel 92 zumindest teilweise aufnehmen können. Die Rastausnehmungen 96 sind in dem Rastabschnitt 98 der Abgleitkulisse 94 ausgebildet. Neben dem Rastabschnitt 98 beinhaltet die Abgleitkulisse 94 den Anschlagabschnitt 100. Der Anschlagabschnitt 100 ist mit einer glatten/ebenen Oberfläche ausgebildet. Der Rastabschnitt 98 und der Anschlagabschnitt 100 sind durch einen ersten Anschlag 102 und einen zweiten Anschlag 104, welche von der Abgleitkulisse 94 hervorstehen, voneinander getrennt. Der erste Anschlag 102 beinhaltet eine Ablauframpe 106.

Die Rastausnehmungen 96 entsprechen, ähnlich wie die Ausnehmungen des Indikatorrastrings 84, definierten Winkeln, um welche das Werkzeug 8 relativ zu dem Schaft 10 bei einer bestimmten Verdrehung des Bedienelements 12 abgewinkelt werden kann. D.h. wenn das Bedienelement 12 um den definierten Winkel relativ zu dem Handgriffabschnitt 4 verdreht wird, dreht das Kugeldruckelement 88 mit dem Bedienelement 12 mit, bis der definierte Winkel erreicht ist, bei welchem die Kugel 92 aufgrund der Vorspannkraft des Federelements 90 in die entsprechende Rastausnehmung 96 der Abgleitkulisse 94 greift. Das zusätzliche Kugeldruckelement 88 ermöglicht folglich ein haptisches Feedback für den Verwender bei der Winkeleinstellung.

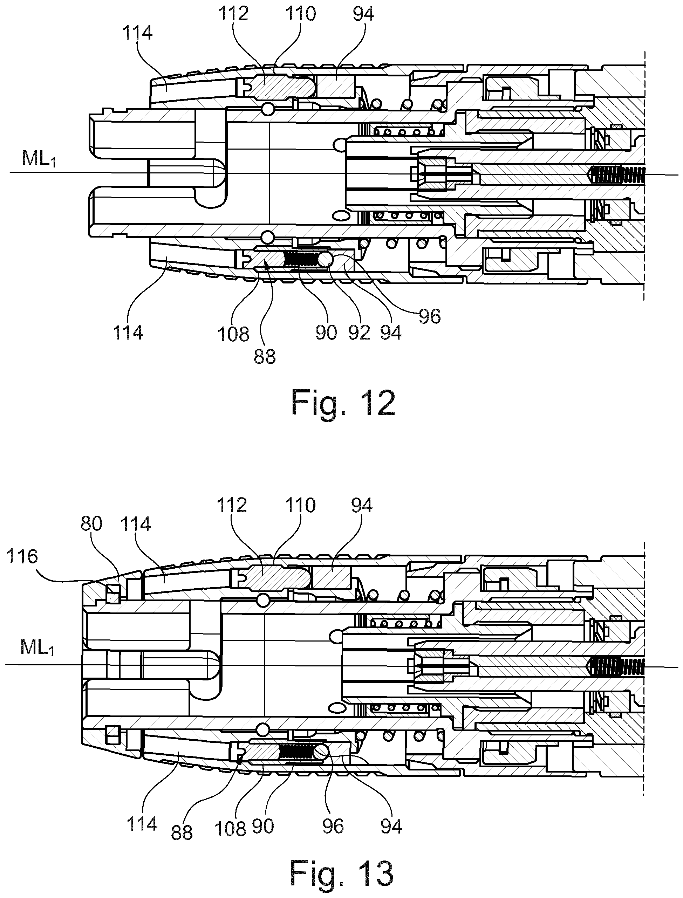

Fig. 12 zeigt eine Schnittansicht des Motorhandstücks 2 in einem Justierungszustand/Montagezustand. Das Bedienelement 12 beinhaltet ein erstes Einstellgewinde 108, welches das Kugeldruckelement 88 aufnimmt. Weiterhin beinhaltet das Bedienelement 12 ein zweites Einstellgewinde 110, welches einen Anschlagstift 112 aufnimmt. Das erste Einstellgewinde 108 ist so positioniert, dass das Kugeldruckelement 88 den Rastabschnitt 98 der Abgleitkulisse 94 kontaktiert. Das zweite Einstellgewinde 110 ist so positioniert, dass der Anschlagstift 112 den Anschlagabschnitt 100 kontaktiert. Das erste Einstellgewinde 108 und das zweite Einstellgewinde 110 erstrecken sich längs zu einer Motorhandstücklängsachse ML1. In der hier dargestellten Ausführungsform erstrecken sich das erste Einstellgewinde 108 das zweite Einstellgewinde 110 parallel zu der Motorhandstücklängsachse ML1. Das erste Einstellgewinde 108 und das zweite Einstellgewinde 110 sind diametral gegenüberliegend bezogen auf die Motorhandstücklängsachse ML1 positioniert.

In einer distalen Verlängerung des ersten Einstellgewindes 108 und des zweiten Einstellgewindes 110 ist jeweils eine Einstellbohrung 114 ausgebildet. Durch die Einstellbohrung 114 kann das Kugeldruckelement 88 bzw. der Anschlagstift 112 justiert werden und mit einem vorbestimmten Drehmoment in Kontakt mit der Abgleitkulisse 94 gebracht werden. Konkret kann durch die Einstellbohrung ein Schraubenzieher oder dergleichen in Kontakt mit dem Anschlagstift 112 und/oder dem Kugeldruckelement 88 gebracht werden und der Anschlagstift 112 und/oder das Kugeldruckelement 88 in einem montierten Zustand in einer Richtung der Motorhandstücklängserstreckung, vorzugsweise in einer Richtung der Motorhandstücklängsachse, justiert werden.

Die Einstellbohrungen 114 sind jeweils hin zu der Motorhandstücklängsachse ML1 geneigt. Durch die Einstellbohrungen 114 kann (nach der Justierung) ein Klebstoff auf den Anschlagstift 112 und das Kugeldruckelement 88 appliziert werden. Auf diese Weise können der Anschlagstift 112 und das Kugeldruckelement 88 in dem justierten Zustand fixiert werden.

Fig. 13 zeigt eine Schnittansicht des Motorhandstücks 2 in einem montierten Zustand. In dem montierten Zustand verschließt die Indikatorhülse/der Indikatorring/Rastring 80 die Einstellbohrung 114 in distaler Richtung. Der Indikatorring/Rastring 80 ist mit einem Sprengring 116 auf dem Motorhandstück 2 vorzugsweise so fixiert, dass er nur mittels Werkzeug entfernt werden kann. Auf diese Weise kann verhindert werden, dass eine Justierung des Anschlagstifts 112 und/oder des Kugeldruckelements 88 durch einen unautorisierten Benutzer manipuliert/verstellt wird.

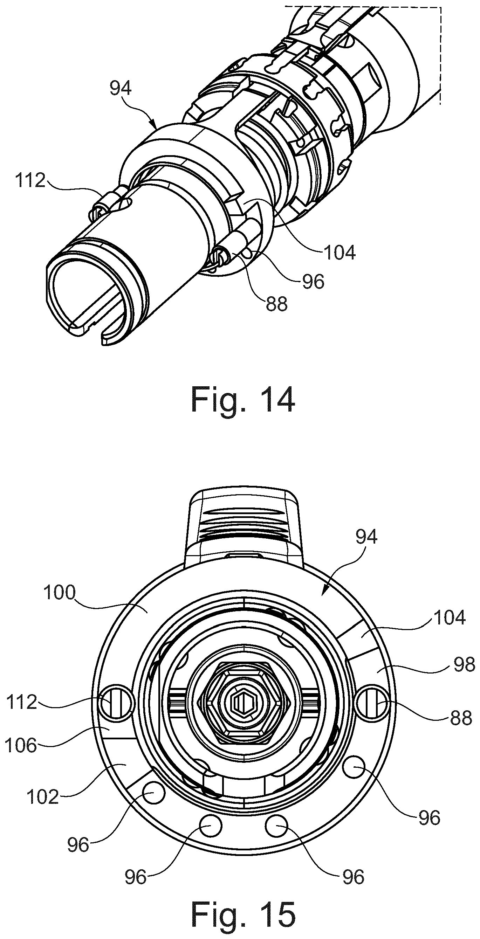

Fig. 14 und Fig. 15 zeigt zur Veranschaulichung das Motorhandstück 2 ohne das Bedienelement 12. Der Anschlagstift 112 und das Kugeldruckelement 88 sind auf dem Durchmesser der Abgleitkulisse 94 angeordnet. Anders ausgedrückt sind das Kugeldruckelement 88 und der Anschlagstift 112 so angeordnet, dass ihr Abstand maximal ist. In nochmals anderen Worten sind das Kugeldruckelement 88 und der Anschlagstift 112 diametral gegenüberliegend angeordnet. Die Anordnung der Rastausnehmungen 96 auf dem Rastabschnitt 98 der Abgleitkulisse 94 entspricht den vordefinierten Winkeln des Werkzeugs 8. Die Anordnung muss hierbei explizit nicht gleichmäßig über den Rastabschnitt 98 der Abgleitkulisse 94 verteilt sein.

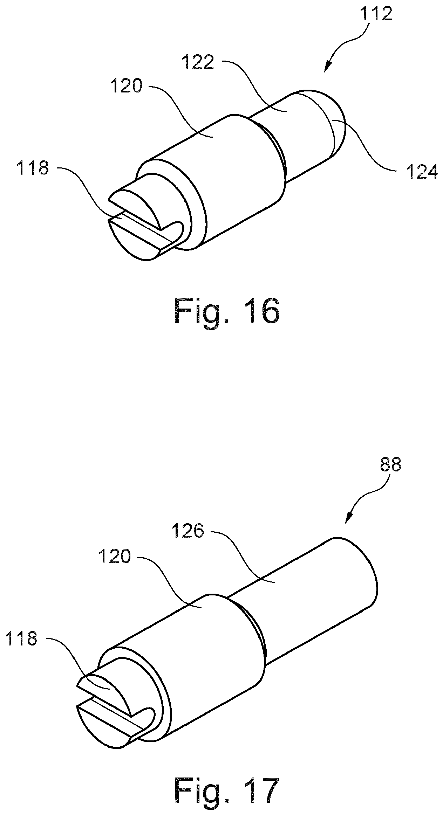

Fig. 16 ist eine Detailansicht des Anschlagstifts 112. Der Anschlagstift 112 beinhaltet einen Schraubenkopfantrieb 118, welcher in der hier dargestellten Ausführungsform als Schlitzschraubenkopfantrieb ausgebildet ist. Der Anschlagstift beinhaltet weiterhin ein Außengewinde 120, das vorgesehen und ausgebildet ist, in das Einstellgewinde 110 des Bedienelements 12 einzugreifen. An einem von dem Schraubenkopfantrieb 118 abgewandten Endabschnitt des Anschlagstifts 112 ist das Anschlagelement 122 ausgebildet. Das Anschlagelement 122 hat im Wesentlichen eine zylindrische Form. Weiterhin beinhaltet der Anschlagstift 112 den Abgleitabschnitt 124, der vorgesehen und ausgebildet ist, an der Abgleitkulisse 94 abzugleiten. Der Abgleitabschnitt 124 ist kugelförmig ausgebildet, um eine Kontaktfläche zwischen Abgleitabschnitt 124 und Abgleitkulisse 94 zu minimieren.

Fig. 17 ist eine Detailansicht des Kugeldruckelements 88. Das Kugeldruckelement 88 beinhaltet einen Schraubenkopfantrieb 118, welcher in der hier dargestellten Ausführungsform als Schlitzschraubenkopfantrieb ausgebildet ist. Der Anschlagstift beinhaltet weiterhin ein Außengewinde 120 das vorgesehen und ausgebildet ist, in das Einstellgewinde 110 des Bedienelements 12 einzugreifen. An einem von dem Schraubenkopfantrieb 118 abgewandten Endabschnitt des Kugeldruckelements 88 ist ein Federabschnitt 126 ausgebildet. Der Federabschnitt 126 ist als Hohlzylinder ausgebildet, in dessen Inneren das Federelement 90 und die Kugel 92 geführt sind (vgl. Fig. 10, Fig. 12 und Fig. 13). Optional kann der Schraubenkopfantrieb 118 des Kugeldruckelements 88 als Schraubstempel ausgebildet sein, über welchen eine Vorspannkraft auf das Federelement 90 eingestellt werden kann.

In einer alternativen Ausführungsform kann ein einziger Schraubstift ausgebildet sein, welcher sowohl die Funktion des Anschlagstift 112 als auch des Kugeldruckelement 88 übernimmt.

In einer weiteren alternativen Ausführungsform kann die Abgleitkulisse 94 mit ausschließlich einem von dem ersten Anschlag 102 oder dem zweiten Anschlag 104 ausgebildet sein.

### Bezugszeichenliste

- 1: Handinstrument
- 2: Motorhandstück
- 4: Handgriffabschnitt
- 6: Antriebseinheit
- 8: Werkzeug
- 10: Schaft
- 12: Bedienelement
- 14: Verstellstift
- 16: Axialnut
- 18: Drehübertragungshülse
- 20: proximaler Schaftabschnitt
- 22: distaler Schaftabschnitt
- 24, 26: angestellte Stirnseite
- 28: erste Kupplungsvorrichtung
- 30: zweite Kupplungsvorrichtung
- 32: Werkzeugkopf / Effektor
- 34: Werkzeugschaft
- 36: Wälzlagereinheit
- 38: Antriebswelle
- 40: Wälzlager
- 42: Lagergehäuse
- 44: Axialsicherungsnut
- 46: Verriegelungskugel
- 48: Raststift
- 50: Außenrohr
- 52: Hohlrad
- 54: Innenverzahnung
- 56: Ritzel
- 58: Außenverzahnung
- 60: Sicherungsbuchse
- 62: Hohlwelle
- 63: Kante
- 64: Aufnahmebohrung
- 66: Wälzlager
- 68: Verstellbuchse
- 70: Silikonschlauch
- 72: Raststift-Aufnahmevertiefung
- 74: Vorspannelement
- 76: Rastvorsprung
- 78: Indikator
- 80: Indikatorhülse
- 82: Verriegelungsschieber
- 83: Zapfenabschnitt
- 84: Indikatorrastring
- 86: Federelement
- 88: Kugeldruckelement
- 90: Federelement
- 92: Kugel
- 94: Abgleitkulisse/Kalottenrastring
- 96: Rastausnehmung
- 98: Rastabschnitt
- 100: Anschlagabschnitt
- 102: erster Anschlag
- 104: zweiter Anschlag
- 106: Ablauframpe
- 108: erstes Einstellgewinde
- 110: zweites Einstellgewinde
- 112: Anschlagstift
- 114: Einstellbohrung
- 116: Sprengring
- 118: Schraubenkopfantrieb
- 120: Außengewinde
- 122: Anschlagelement
- 124: Abgleitabschnitt
- 126: Federabschnitt
- ML1: Motorhandstücklängsachse

## Patentansprüche

1. Medizinisches Motorhandstück (2) zum Antreiben eines distalen Endeffektors (32), mit
einem Handgriffabschnitt (4),
einem vorzugsweise hülsenförmigen Bedienelement (12), das um eine Handgrifflängsachse drehbar an einem distalen Endabschnitt des Handgriffabschnitts (4) gehalten ist und mit dem Endeffektor (32) derart koppelbar oder gekoppelt ist, um eine Drehbewegung des Bedienelements (12) in eine Bewegung des Endeffektors (32) zu transformieren oder ein Funktion am Endeffektor (32) zu bewirken und
eine Rast- und/oder Anschlageinheit, die dafür vorgesehen und ausgebildet ist, die Drehbewegung des Bedienelements (12) an Endpositionen zu begrenzen und/oder einen Drehbewegungswiderstand auf das Bedienelement (12) in wenigstens einer Drehzwischenposition zu erhöhen,
**dadurch gekennzeichnet, dass**
die Rast- und/oder Anschlageinheit zumindest einen Schraubstift (88; 112), welcher längs zur Handgrifflängsachse sich erstreckend in ein Einstellgewinde (108, 110) des Bedienelements (12)
eingeschraubt ist und eine Abgleitkulisse (94) aufweist, die drehfest am Handgriffabschnitt (4) gehalten und mit dem zumindest einen Schraubstift (88; 112) in Gleiteingriff bringbar ist.

2. Medizinisches Motorhandstück (2) nach Anspruch 1, **dadurch gekennzeichnet, dass** es sich bei dem zumindest einen Schraubstift (88; 112) um eine Mehrzahl von Schraubstiften (88; 112) handelt, welche als Anschlagstift (112) und/oder als Kugeldruckstück (88) ausgebildet sind, wobei eine Anzahl an Anschlagstiften (112) vorzugsweise einer Anzahl an Kugeldruckstücken (88) entspricht.

3. Medizinisches Motorhandstück (2) nach Anspruch 2, **dadurch gekennzeichnet, dass** das Kugeldrückstück (88) eine federvorgespannte Kugel (92) beinhaltet, wobei eine Vorspannkraft des Kugeldrückstücks (88) über das Einstellgewinde (110) einstellbar ist.

4. Medizinisches Motorhandstück (2) nach einem der Ansprüche 1 bis 3, **dadurch gekennzeichnet, dass** es sich es bei dem zumindest einen Schraubstift (88; 112) um genau zwei Schraubstifte (88; 112) handelt, welche auf einem konstanten Radius um eine Mittelfaser (ML1) des Motorhandstücks (2), vorzugsweise gegenüberliegend auf einem Kreisdurchmesser, angeordnet sind.

5. Medizinisches Motorhandstück (2) nach einem der Ansprüche 1 bis 4, **dadurch gekennzeichnet, dass** sich an das Einstellgewinde (108, 120) in distaler Richtung eine Einstellbohrung (114) anschließt.

6. Medizinisches Motorhandstück (2) nach Anspruch 5, **dadurch gekennzeichnet, dass** die Einstellbohrung (114) zwischen einem distalen Endabschnitt des Einstellgewindes und einem distalen Endabschnitt des Bedienelements (12), vorzugsweise einer distalen Endfläche des Bedienelements (12), erstreckt.

7. Medizinisches Motorhandstück (2) nach Anspruch 5 oder 6, **dadurch gekennzeichnet, dass** die Einstellbohrung (114) in distaler Richtung zu der Mittelfaser (ML1) des Motorhandstücks (2) hin geneigt ist.

8. Medizinisches Motorhandstück (2) nach Anspruch 7, **dadurch gekennzeichnet, dass** die Einstellbohrung (114) in einem Winkel kleiner als 10 ° zu der Mittelfaser (ML1) geneigt ist.

9. Medizinisches Motorhandstück (2) nach einem der Ansprüche 5 bis 8, **dadurch gekennzeichnet, dass** die Einstellbohrung (114) distal durch einen Rastring (80) verschlossen ist.

10. Medizinisches Motorhandstück (2) nach einem der Ansprüche 1 bis 6, **dadurch gekennzeichnet, dass** der zumindest eine Schraubstift (88; 112) mittels Klebstoff fixiert ist.

11. Medizinisches Motorhandstück (2) nach einem der Ansprüche 1 bis 10, **dadurch gekennzeichnet, dass** die Abgleitkulisse (94) ringförmig ausgebildet ist und auf einer distalen Stirnseite einen ersten Abschnitt (98) mit Rastlöchern (96) und einen zweiten glatten Abschnitt (100) beinhaltet.

12. Medizinisches Motorhandstück (2) nach Anspruch 11, **dadurch gekennzeichnet, dass** der erste Abschnitt (98) und der zweite Abschnitt (100) durch Anschläge (102, 104) getrennt sind welche in distaler Richtung von der Abgleitkulisse (94) hervorstehen.

13. Medizinisches Handinstrument (1) mit
- einem medizinischen Motorhandstück (2) nach einem der Ansprüche 1 bis 9
- einem Werkzeugschaft (10)
- einem Effektor (32).

14. Medizinisches Motorhandstück (2) nach Anspruch 13, **dadurch gekennzeichnet, dass** der Endeffektor (32) relativ zu dem Werkzeugschaft (10) abwinkelbar ist.
